(19) Europäisches Patentamt
European Patent Office
Office européen des brevets

(11) Publication number: **0 150 105 A2**

(12) # EUROPEAN PATENT APPLICATION

(21) Application number: 85300328.3

(22) Date of filing: 17.01.85

(51) Int. Cl.[4]: C 07 C 2/02
B 01 J 29/28

(30) Priority: 24.01.84 GB 8401832

(43) Date of publication of application:
31.07.85 Bulletin 85/31

(84) Designated Contracting States:
BE DE FR GB IT NL SE

(71) Applicant: The British Petroleum Company p.l.c.
Britannic House Moor Lane
London EC2Y 9BU(GB)

(72) Inventor: Barri, Sami Ali Ibrahim The British
Petr.Comp.plc
Chertsey Road Sunbury-on-Thames
Middlesex, TW16 7LN(GB)

(72) Inventor: Hall, Antony Harold Patrick The British
Petr.Comp
Chertsey Road Sunbury-on-Thames
Middlesex, TW16 7LN(GB)

(72) Inventor: Young, Dennis The British Petroleum
Company plc
Chertsey Road Sunbury-on-Thames
Middlesex, TW16 7LN(GB)

(74) Representative: Krishnan, Suryanarayana
Kalyana et al,
BP INTERNATIONAL LIMITED Patents Division Chertsey
Road
Sunbury-on-Thames Middlesex TW16 7LN(GB)

(54) **Production of transport fuels from light olefins.**

(57) The present invention relates to a process for the conversion of a gaseous hydrocarbon feedstock rich in light olefins into a liquid product which is a fuel or is capable of being converted into a liquid fuel. The feedstock is brought into contact with a catalyst derived from a tectometallosilicate e.g. Theta-1 zeolite at a temperature from 100-500°C.

EP 0 150 105 A2

PRODUCTION OF TRANSPORT FUELS FROM LIGHT OLEFINS

The present invention relates to a process for the production of transport fuels from light olefins in the presence of tectometallosilicates, such as zeolites.

Zeolites are well known natural and synthetic compositions. Many of them have been demonstrated to have catalytic properties for various types of hydrocarbon conversion and related reactions. Zeolites can be defined as ordered porous crystalline aluminosilicates having a framework structure consisting of a rigid regular three dimensional network of $SiO_4$ and $AlO_4$ tetrahedra in which the tetrahedra are cross-linked by sharing the oxygen atoms and which are sufficiently open to accommodate at least water molecules. Such structures generally contain a regular array of small voids interconnected by channels or pores. The dimensions of the voids and channels can range from those of water to those of quite large molecules. For a given framework structure, the dimensions of the voids and channels are limited to a small number of values, which can vary from structure to structure. Thus these structures are capable of absorbing molecules of certain dimensions while rejecting those of dimensions larger than a critical value which varies with structure. This has led to zeolites being used as molecular sieves. Zeolites belong to a class of materials that can be termed tectoaluminosilicates which comprise (in addition to zeolites) felspars and felspathoids. All oxygen atoms are shared, thus the ratio of total aluminium and silicon atoms to oxygen atoms is 1:2. The inclusion of aluminium in the framework leads to a net

negative charge which is balanced by the inclusion in the crystal of an electrochemical equivalence of cations, for example alkali metal, alkaline earth metal, hydrogen or ammonium cations or mixtures thereof. This can be expressed by a formula in which the ratio of Al to the number of the various cations such as Ca/2, Sr/2, Na, K, Li or generally M/n (where M is the cation and n is the formal oxidation state of the cation) is equal to unity. Additionally in zeolites, but not in felspars and some felspathoids, the framework is sufficiently open to accommodate water molecules as well as cations. This enables these cations to be exchanged in their entirety or partially by other cations using ion-exchange techniques in a conventional manner. These materials can exhibit specific affinities for specific cations and can thus be used as selective ion-exchangers. By means of ion-exchange, it is possible to vary the size of the pores in a given crystalline zeolite material, modifying its molecular sieve properties. Also by means of ion-exchange the catalytic properties of these materials can be altered. In addition to the framework and charge-compensating cations, zeolites can contain other materials such as water and organic molecules, (hydrated) salts and oxides of e.g. Na, Al and Si introduced during synthesis, or formed or added during subsequent treatments. Zeolites are best characterised according to framework structure type, i.e. on the topology of the framework, irrespective of composition, distribution of different tetrahedral atoms, cell dimensions and symmetry. A code consisting of three capital letters has been adopted for each known structure type following the recommendations by IUPAC on zeolite nomenclature ("Chemical Nomenclature, and Formulation of compositions, of Synthetic and Natural Zeolites", IUPAC yellow booklet, 1978) and a compilation of 38 known zeolite structure types has been published by The Structure Commission of the International Zeolite Association ("Atlas of Zeolite Structure Types", by Meier, W.M. and Olsen, D.H. (1978), distributed by Polycrystal Book Service, Pittsburgh, Pa, USA). In addition to the groups classified by known structure type, there is a further group of crystalline zeolite materials whose X-ray

diffraction patterns, sorption, ion-exchange and related properties indicate that they do not have known structure types but appear to have new, as yet undetermined structure types. An example of such a material is the novel porous crystalline aluminosilicate designated Theta-1 and described in our published European patent specification No. 0057049.

Zeolites (and other tectoaluminosilicates) belong to a larger class of materials that can be termed tectometallosilicates which can be defined in the same way as tectoaluminosilicates except that the aluminium is replaced by a range of elements, which includes, it is claimed, Ti, Zr, V, Cr, Mo, Mn, Fe, Co, Rh, Ni, Zn, B, Al, Ga, Ge, Sn, As and Sb, but in some cases the claimed materials have not been well characterised. In some cases (where the element has the same formal oxidation state as Si i.e. +4) the resultant framework is electroneutral and the resultant materials resemble crystalline silicas. In other cases there is a resultant framework negative charge which must be compensated by cations as in tectoaluminosilicates. In some cases the materials have porous frameworks like those of zeolites or porous crystalline silicas which they therefore resemble.

Use of aluminosilicates, in particular the use of MFI-type aluminosilicate for production of transport fuels such as gasoline from hydrocarbon products rich in olefins is known (e.g. US 3,775,501 3,960,978). However, in the known processes the MFI-type aluminosilicate has to be modified by means of activating or catalysing agents in order to achieve the desired degree of selective conversion of the appropriate light olefins.

Generally, it is the formation of aromatics and paraffins over un-modified MFI-type aluminosilicates that has to be suppressed.

Theta-1-type aluminosilicates, as described in our European Patent Publication No. 0057049 are expected to have similar catalytic properties to MFI-type aluminosilicates because of very similar physico-chemical properties. MFI- and Theta-1-type aluminosilicates can be prepared with very similar $SiO_2$:$Al_2O_3$ ratios and with near identical shape-selective sorption characteristics.

For example, they both adsorb and desorb xylenes. They both can be prepared from very similar starting gels (including the same template such as diethanolamine) subjected to identical digestion parameters, see for example our European Patent Publication No 0057049. Thus it could be reasonably expected that conversion of light olefins over an un-modified hydrogen-form of Theta-1 aluminosilicate should lead to a product rich in aromatics if exposed to conditions under which the un-modified hydrogen-form of MFI-type aluminosilicate leads to the formation of aromatics.

Surprisingly, it has now been found that the hydrogen-form of Theta-1 type aluminosilicates and other tectometallosilicates of similar structure can be used without further modification to convert light olefins to transport fuels such as gasoline.

Accordingly, the present invention is a process for the conversion of a gaseous hydrocarbon feedstock rich in light olefins into a liquid product which is a fuel or is capable of being converted into a liquid fuel by bringing the feedstock into contact with a catalyst derived from a tectometallosilicate at an elevated temperature.

The tectometallosilicate has the following composition in terms of the mole ratios of the oxides:

$$(0.9 \pm 0.25)M_{\frac{4-m}{n}} : XO_2 : xSiO_2 : yH_2O:zQ$$

wherein M is at least one cation having a valence n, X is one or more of the metals selected from Al, Ga, Zn, Fe, Cr and B, m is the valency of the metal X in the metal oxide $XO_2$, Q is a template used in the synthesis of the tectometallosilicate, x is at least 10, y/x is from 0 to 5, and z/x is 0-20, and the tectometallosilicate in its organo-free hydrogen-form has an X-ray diffraction pattern substantially as set forth in Table A of this specification.

Thus the tectometallosilicates used in the present invention have a Theta-1-type structure as described in our European Patent Specification No. 0057049.

It is preferable to have the Theta-1 type tectometallosilicate in the organic-free hydrogen form in the catalyst.

By the term "organic-free hydrogen-form" is meant here and throughout this specification that the tectometallosilicate has been rendered substantially free of organic material introduced during synthesis or subsequent treatments, and the cation M is hydrogen. The tectometallosilicates prepared from organic bases contain organics as synthesised and these can suitably be removed by calcination in air. The tectometallosilicates prepared from ammonia do not contain organics as synthesised. Therefore, in such a case, an organics removal step such as calcination in air is not essential.

The $H_2O$ content "y" of the tectometallosilicate is the water of hydration and will depend, within the ratios set out above, upon the conditions under which it is dried, calcined, subjected to further aqueous treatments or combinations thereof after synthesis. The $H_2O$ content "y" does not include water which may be notionally present when the cation M is hydrogen.

The 'template' is a chemical agent, e.g. an organic or inorganic base which encourages crystallisation to proceed towards a particular framework structure or structures.

The content "z" of template "Q" in the tectometallosilicate will also depend upon the conditions under which the tectometallosilicate is washed, calcined or subjected to further aqueous treatments or combinations thereof after synthesis, and also on the synthesis parameters of the gallosilicate, particularly the proportion of Q present in the original hydrogel. The molar ratio of the template Q to silica, i.e. z/x is preferably 0-5 in the tectometallosilicate as synthesised. The template content is usually highest for the "parent" tectometallosilicate. Complete removal of the template, if present, is usually only possible by thermal or oxidative degradation or both.

By the "parent" tectometallosilicate is meant throughout this specification the product of synthesis and washing and optionally drying.

The cation M in the tectometallosilicate may be selected from $H^+$, ammonium, alkali metal cations, alkaline earth metal cations,

organic nitrogen containing cations, aluminium cation, gallium cation and mixtures thereof.

The cations present in the tectometallosilicate may be replaced using conventional ion exchange techniques either wholly or partially by other cations e.g. hydrogen ions or metal cations.

The organic-free hydrogen-form of the tectometallosilicate may be produced by known methods such as exchange with hydrogen ions or with ammonium cations followed by one or more calcinations or a combination of the two followed by one or more calcination stages, if the tectometallosilicate still contained ammonium ions.

The metal X in the metal oxide $XO_2$ is preferably Al, Ga, Zn, Fe, B or Cr. In the tectometallosilicate the molar ratio of silica to metal X is preferably from 20:1 to 500:1.

The tectometallosilicates according to the present invention have in their organic-free hydrogen form an X-ray diffraction pattern shown in Table A below. The specific values in the Table are determined using copper K-alpha radiation and a computer step scan.

The peak heights, I, and their position as a function of 2-theta, where theta is the Bragg angle, are read from the spectrometer output. From this output the relative intensities 100 x $I/I_o$, where $I_o$ is the intensity of the strongest peak, and d the interplanar spacing in A, corresponding to the recorded peaks are calculated.

It will be understood by those skilled in the art that the X-ray diffration pattern of tectometallosilicates may vary in the values of $I/I_o$ and the d-spacing depending for example upon whether the sample being examined is calcined or uncalcined, upon the temperature of calcination, upon the nature of the cation present in the tectometallosilicate, the mole ratio of silica to metal oxide, and the particle size of the tectometallosilicate.

The tectometallosilicate is suitably produced by mixing a source of silica, a source of gallia, a source of alkali metal(s), water and an organic or inorganic nitrogen containing base until a homogeneous gel is formed and crystallising the gel at a temperature above 70°C.

TABLE A

| 2-theta (degrees) | d-spacing (Angstroms) | Relative Intensity 100 x $I/I_0$ |
|---|---|---|
| 8.06 ± 0.2 | 11.25 - 10.70 | 50 to 100 |
| 10.06 ± 0.2 | 9.01 - 8.63 | 5 to 30 |
| 12.69 ± 0.2 | 7.09 - 6.87 | 10 to 30 |
| 16.28 ± 0.2 | 5.51 - 5.38 | 5 to 15 |
| 19.40 ± 0.2 | 4.62 - 4.53 | 5 to 15 |
| 20.26 ± 0.2 | 4.43 - 4.34 | 50 to 100 |
| 24.11 ± 0.2 | 3.72 - 3.66 | 50 to 100 |
| 24.52 ± 0.2 | 3.66 - 3.60 | 30 to 90 |
| 25.65 ± 0.2 | 3.50 - 3.45 | 15 to 45 |

scanned up to 2 theta = 32

The Theta-1 type tectometallosilicates used in the present invention may be bound in a suitable binding material using methods and materials well known to those skilled in the art.

The hydrocarbon feedstock rich in light olefins suitably contains a major proportion of $C_2$-$C_6$ olefins. The feedstock may be diluted with fluids inert under the reaction conditions such as nitrogen or light paraffins.

The feedstock is suitably brought into contact in the vapour or liquid phase with the tectometallosilicate depending upon the reaction conditions.

The conversion of the hydrocarbon feedstock e.g. olefins to a product that is liquid at ambient temperatures and pressures, and which is a fuel or is capable of being converted into a liquid fuel is suitably carried out using the hydrogen form of the alumino-tectometallosilicate especially the hydrogen form of the aluminosilicate. It is preferable to conduct the conversion at a temperature from 100-500°C.

The conversion is preferably carried out at a reaction pressure of 1-1000 bar preferably 1-100 bar and a weight hourly space velocity of 0.01-100, preferably from 0.1 to 20.

Reference is made to a liquid product capable of being converted into a liquid fuel because in some cases it may be desirable to hydrogenate the reaction product to make it into a more desirable transport fuel such as gasoline or diesel, or to improve its blending characteristics with such fuels.

A principal advantage of the present invention is that the conversion can be carried out without adding any modifying components to the tectometallosilicate. Moreover, the process results in a very high selectivity to $C_{5+}$ olefins which fall within the gasoline and diesel boiling ranges. A useful by-product of the reaction can be aromatic hydrocarbons containing 6-9 carbon atoms which could be used as high octane gasoline blending components or as petrochemical feedstock. Significantly, the selectivity of the present process to undesirable $C_1$-$C_4$ by-products is very low.

The process can be combined with a paraffin dehydrogenation process to give a combined paraffins to hydrocarbon liquids process.

The present invention is further illustrated with reference to the following Examples.

EXAMPLES

A. Catalyst Preparation

(i) A solution was prepared from a mixture of sodium aluminate (12.6g), sodium hydroxide (6.65g) and water (140g). Diethanolamine (DEA, 180g) was melted and added to the solution and the resultant solution "X" was stirred and maintained at room temperature. 500g of a commercial silica sol., 'Ludox AS40' (Reg. Trade Mark) which contains 40% by weight of silica, was further diluted with (354g) water to form solution "Y". Thereafter solution Y was added dropwise to solution X over a period of 15 minutes with vigorous stirring. Stirring was continued for a further 20 minutes and then the resultant gel which had the composition:

$2.64\ Na_2O : 31.8\ (DEA) : Al_2O_3 : 61.9\ SiO_2 : 819\ H_2O$ was transferred to an oven and crystallised with agitation at 175°C for 48 hours in a revolving stainless steel pressure vessel. The product was filtered, washed and dried at 90°C.

(ii) The dried product was transferred to an oven and heated to 550°C over 4 hours then maintained at 550°C for 60 hours then cooled. The calcined product was then refluxed in 10 volume per cent aqueous nitric acid for 2.5 hours, filtered, washed then refluxed in 0.67 M aqueous ammonium nitrate for 4 hours. In both refluxes the solution: zeolite weight ratio was 6:1. The product was filtered, washed, dried at 90°C then transferred to an oven, heated to 550°C over 4 hours, maintained at 550°C for 16 hours then cooled. The X-ray powder diffraction pattern of the resultant material is shown in Table 1 and shows the product to be good crystalline Theta-1. The resultant material was mixed with twice its weight of Ludox AS40 and just sufficient distilled water to wet the mixture. The resulting slurry was dried at 90°C and the resultant cake was broken and sieved to 12 to 30 mesh BSS.

Table 1

| 2-theta (degrees) | d (Angstroms) | Relative Intensity $I/I_o$ (%) |
|---|---|---|
| 8.15 | 10.85 | 100 |
| 10.15 | 8.72 | 17 |
| 12.77 | 6.93 | 17 |
| 16.35 | 5.42 | 10 |
| 19.41 | 4.57 | 11 |
| 20.34 | 4.37 | 84 |
| 24.19 | 3.68 | 88 |
| 24.63 | 3.61 | 54 |
| 25.72 | 3.46 | 28 |
| 35.59 | 2.52 | 21 |

Scanned up to 2-theta = 36° and $I/I_o$% = 10 or more

B. Conversion of Light Olefins to a source of Fuels

(i) 6 ml of the catalyst prepared in Section A was transferred to a reactor where it was pretreated with flowing dry hydrogen at 650°C for 4 hours, cooled to 550°C under flowing dry nitrogen and retreated with flowing dry air for 4 hours. The catalyst was then cooled to 400°C in flowing dry nitrogen.

(ii) Propylene was then passed over the catalyst so formed under the conditions in Table 2. Products of the reaction are listed in Table 2.

Comparative Test

(i) The method of Section A (i) was followed closely except that the gel composition was:

$$2.40\ Na_2O : 18.6\ DEA : Al_2O_3 : 36.5\ SiO_2 : 545\ H_2O$$

The gel was crystallised at 145°C for 96 hours. The product was filtered, washed and dried as described in Section A (i).

(ii) The dried product was converted to a catalyst in the same manner as in Section A (ii) above. The X-ray powder diffraction pattern of the calcined powder was consistent with that of crystalline MFI-type aluminosilicate.

A 6 ml (approx.) sample of the catalyst prepared in the Comparative Test above was pretreated as in Example B (i) above. The catalyst was then contacted with propylene under the conditions shown in Table 2 below. The reaction products are also shown in Table 2.

<u>Table 2</u>

<table>
<tr><th>Catalyst</th><th colspan="2">Example</th><th colspan="2">Comparative Test</th></tr>
<tr><td>T (°C) (1)</td><td colspan="2">403</td><td colspan="2">430</td></tr>
<tr><td>Pressure (MPa)</td><td colspan="2">0.1</td><td colspan="2">0.1</td></tr>
<tr><td>ct (sec) (2)</td><td colspan="2">2.16</td><td colspan="2">1.52</td></tr>
<tr><td>Hours on Stream</td><td colspan="2">0.5 to 1.5</td><td colspan="2">0.5 to 1.5</td></tr>
<tr><td>Yield (wt%)</td><td colspan="2"></td><td colspan="2"></td></tr>
<tr><td>$H_2$</td><td colspan="2">less than 0.1</td><td colspan="2">1.0</td></tr>
<tr><td>$C_1$</td><td colspan="2">less than 0.1</td><td colspan="2">3.1</td></tr>
<tr><td>$C_2$ olefins</td><td colspan="2">2.5</td><td colspan="2">0.6</td></tr>
<tr><td>$C_2$ paraffins</td><td colspan="2">0.1</td><td colspan="2">2.8</td></tr>
<tr><td>$C_3$ olefins</td><td colspan="2">10.9</td><td colspan="2">0.8</td></tr>
<tr><td>$C_3$ paraffins</td><td colspan="2">8.8</td><td colspan="2">28.7</td></tr>
<tr><td>$C_4$ olefins</td><td colspan="2">8.0</td><td colspan="2">less than 0.1</td></tr>
<tr><td>$C_4$ paraffins</td><td colspan="2">6.6</td><td colspan="2">11.4</td></tr>
<tr><td>$C_{5+}$ PNO (3)</td><td>45.1</td><td rowspan="2">63</td><td>3.0</td><td rowspan="2">50</td></tr>
<tr><td>$C_{6+}$ ARO (4)</td><td>17.9</td><td>47.0</td></tr>
<tr><td>Selectivity (5)</td><td colspan="2">78</td><td colspan="2">50</td></tr>
</table>

(1) Average bed temperature

(2) Superficial contact time = $\dfrac{\text{volume of catalyst bed}}{\text{volume flowrate of feed @ T\&P}}$

(3) $C_{5+}$ PNO = Paraffins, Napthlenes, Olefins (Primarily olefinic acyclics)

(4) $C_{6+}$ ARO = Aromatics, (Primarily $C_6$-$C_9$ aromatics)

(5) Selectivity = $\dfrac{\text{Yield } (C_{5+}) \times 100}{100 - \text{Yield } (C_3= + C_4=)}$

Claims:

1. A process for the conversion of a gaseous hydrocarbon feedstock rich in light olefins into a liquid product which is a fuel or is capable of being converted into a liquid fuel by bringing the feedstock into contact with a catalyst derived from a tectometallosilicate at an elevated temperature.

2. A process according to claim 1 wherein the tectometallosilicate has the following composition in terms of the mole ratios of the oxides:

$$(0.9 \pm 0.25)M_{\frac{4-m}{n}} : XO_2 : xSiO_2 : yH_2O:zQ$$

wherein M is at least one cation having a valence n, X is one or more of the metals selected from Al, Ga, Zn, Fe, Cr and B, m is the valency of the metal X in the metal oxide $XO_2$, Q is a template used in the synthesis of the tectometallosilicate, x is at least 10, y/x is from 0 to 5, and z/x is 0-20, and the tectometallosilicate in its organo-free hydrogen-form has an X-ray diffraction pattern substantially as set forth in Table A of this specification.

3. A process according to claim 1 or 2 wherein the hydrocarbon feedstock rich in light olefins comprises a major proportion of $C_2$-$C_6$ olefins.

4. A process according to any one of the preceding claims wherein the conversion is carried out using the hydrogen form of the tectometallosilicate.

5. A process according to any one of the preceding claims wherein the conversion is carried out at a temperature from 100-500°C.

**6.** A process according to any one of the preceding claims wherein the process is carried out at a pressure from 1-1000 bar.

**7.** A process according to any one of the preceding claims wherein the process is carried out at a weight hourly space velocity (WHSV) of 0.01-100.

**8.** A process according to any one of the preceding claims wherein the conversion is carried out without adding any modifying components to the tectometallosilicate.

**9.** A process for the conversion of a gaseous hydrocarbon feedstock rich in light olefins into a liquid product which is a fuel or is capable of being converted into a liquid fuel as claimed herein and as described hereinbefore with reference to the Examples.